# EUROPEAN PATENT SPECIFICATION

(11) **EP 1 093 800 B1**
(45) Date of publication and mention of the grant of the patent: **19.12.2007**
(21) Application number: 00122264.5
(22) Date of filing: 19.10.2000
(51) Int. Cl.: A61K 8/04, A61K 8/42, A61Q 19/00

(54) **Cosmetic composition**
Kosmetische Zubereitung
Composition cosmétique

(30) Priority: 22.10.1999 JP 30141999
(43) Date of publication of application: 25.04.2001
(73) Proprietor: KAO CORPORATION, Chuo-Ku,Tokyo 103 (JP)
(72) Inventor: Hasebe, Keiko, Wakayama-shi, Wakayama 640-8580 (JP); Sata, Juri, Wakayama-shi, Wakayama 640-8580 (JP); Tamura, Yoshinori, Wakayama-shi, Wakayama 640-8580 (JP); Doi, Yasuhiro, Wakayama-shi, Wakayama 640-8580 (JP); Masukawa, Yoshinori, Ichikaimachi, Haga-gun, Tochigi 321-3497 (JP); Tsujimura, Hisashi, Ichikaimachi, Haga-gun, Tochigi 321-3497 (JP)
(74) Representative: HOFFMANN EITLE

(56) References cited:
- EP-A- 0 487 958
- EP-A- 1 013 258
- EP-A- 1 172 083
- EP-A- 1 206 932
- DE-A- 19 751 550
- US-A- 5 344 650
- US-A- 5 863 945

## Description

The present invention relates to the use of a cosmetic composition comprising a dispersion (A) containing the following components (a) and (b):
(a) 5 to 40 % by weight of a dispersoid composed of a particulate amphipathic lipid having at least one hydroxyl and amide group in its molecule, which is solid at 25°C, and which has an average particle diameter of 1 to 150 µm; and
(b) 2 to 55 % by weight of a surfactant in an aqueous medium,
or a cosmetic composition comprising 0.01 to 30 % by weight of a particulate amphipathic lipid having an average particle diameter of 1 to 150 µm, which is solid at 25°C, and which has at least one hydroxyl and amide group in its molecule, in a cosmetic method, wherein said cosmetic composition is applied to a user's body, and wherein said cosmetic composition is not washed off from said user's body after said cosmetic composition has been applied thereto.

The amphipathic lipid as dispersoid is excellent in moisturizing effect and skin roughness-improving effect, and gives users a pleasant feeling upon use.

### BACKGROUND ART

A feature of a healthy skin resides in that it is moistened and flexible. It has heretofore been known that the maintenance of water in the horny layer is important for retaining the healthy skin, and water-soluble components contained in the horny layer, namely, free amino acids, urea, organic acids, inorganic ions, etc., participate in the maintenance of the water. On the other hand, amphipathic lipids, particularly, ceramides are known to exist in the horny layer of a skin and contribute to the barrier function and water-retaining ability thereof. It is also known that the dried state of the skin can be improved by their external application. Therefore, many ceramides or analogues (pseudoceramides) thereof have been produced and incorporated into skin cosmetic compositions. The present applicant has previously developed hair cosmetic compositions comprising a specific amide derivative as the amphipathic lipid (Japanese Patent Application Laid-Open No. 9913/1989).

However, the ceramides or analogues thereof have such features that their melting points are generally high, the crystallinity is high, and the compatibility with other compounds is low, and so they have been difficult to handle, since they have had to be heated and melted at a high temperature upon their incorporation into cosmetic compositions, and techniques of high level have been required to stably mix them with other compounds in the form of liquid crystals or the like. When they have been intended to be incorporated by the conventional technique, their incorporation has been limited to extremely small amounts, resulting in difficulty in incorporating them in amounts sufficient to fully derive their effects. Incidentally, Japanese Patent Application Laid-Open No. 9913/1989 describes only the fact that the amide derivative is obtained as colorless powder, but does not describe anything about the fact that a dispersion, which will be described subsequently, is prepared and incorporated, or the particle diameter of the amide derivative is defined.

It is therefore an object of the present invention to provide the use of a cosmetic composition in a cosmetic method wherein it is not washed off after application, in which an amphipathic lipid represented by a ceramide or an analogue thereof is stably incorporated, excellent moisturizing effect and skin roughness-improving effect are brought about, and a pleasant feeling is given upon use.

DE 197 51 550 A1 relates to a cosmetic composition on an aqueous basis comprising ceramides, a fatty acid and a sterol.

US 5,344,650 describes an emulsion-type aqueous cosmetic composition comprising a mixture of oil and fat, an aqueous medium and an emulsifying agent.

US 5,863,945 relates to specific amide derivatives and external skin care preparations, cosmetic hair care formulations and bath formulations containing the same.

EP 487 958 A1 describes a cosmetic composition comprising an amphiphatic lipid, a nonionic surfactant, an ionic surfactant and an aqueous medium, which is transparent or semi-transparent, and wherein the amphiphatic lipids are stably microdispersed.

EP 1 013 258 A2 describes a dispersion that comprises, as a dispersoid, 5-40 wt.% of an amphiphatic lipid containing at least one hydroxy and amide group in its molecule and a surfactant in an aqueous medium.

EP 1 206 932 A1 relates to cosmetics containing one or more components selected from lipids contained in the stratum corneum and analogues thereof, and terpene components other than menthol. The cosmetics are said to be efficacious in ameliorating chapped skin and roghened skin and have effects of improving skin qualities.

EP 1 172 083 A2 relates to a skin cosmetic composition comprising a hydrogel particle comprising a non-crosslinked hydrogel forming a continuous phase containing an oil component dispersed therein, said particle being dispersed in an aqueous medium.

### DISCLOSURE OF THE INVENTION

The present inventors have found that when a dispersion with an amphipathic lipid dispersed at a high concentration in an aqueous medium containing a certain amount of a surfactant in advance is used, the amphipathic liquid is stably incorporated in the form of particles having a specified particle diameter, thereby providing a cosmetic composition of the type that it is not washed off after applied to a user's body, by which excellent moisturizing effect and skin roughness-improving effect are brought about, and a pleasant feeling is given upon use.

According to the present invention, there is thus provided the use of a cosmetic composition in a cosmetic method, as defined in the claims.

### BEST MODE FOR CARRYING OUT THE INVENTION

The amphipathic lipid of the component (a) used in the dispersion (A) in the present invention and having at least one hydroxyl group and an amide group in its molecule means one of natural ceramides, synthetic ceramides and their analogues (pseudoceramides) obtained by synthesis or the like, which is solid at room temperature (25°C). Examples of such an amphipathic lipid include Ceramide H03 (Sederma Co.), Ceramide II (Sederma Co.), Questamide H (Quest Co.), Ceramide TIC-001 (Takasago Corporation) and Sofcare Ceramide SL-E (Kao Corporation). In the present invention, as the amphipathic lipid, that having a melting point of not less than 30°C is preferred, and that having a melting point of not less than 40°C is more preferred from the viewpoint of stability when a dispersion or the like is prepared. Particularly preferable examples of the ceramide analogues obtained by synthesis include amide derivatives represented by the following general formula (1) and including the above-mentioned Sofcare Ceramide SL-E. 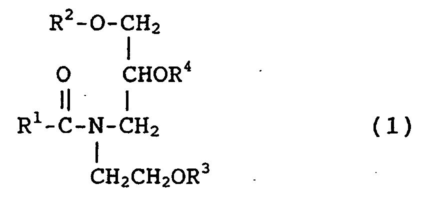 wherein R¹ and R² are the same or different from each other and are, independently, a linear or branched, saturated or unsaturated hydrocarbon group having 7 to 39 carbon atoms, which may be substituted by at least one hydroxyl group, and R³ and R⁴ are the same or different from each other and are, independently, a hydrogen atom, phosphate residue, sulfate residue or sugar residue, with the provide that the molecule has at least one hydroxyl group therein.

In the general formula (1), the hydrocarbon group of R¹ is preferably a linear or branched, saturated or unsaturated hydrocarbon group having 9 to 25 carbon atoms, the hydrocarbon group of R² is preferably a linear or branched, saturated or unsaturated hydrocarbon group having 10 to 26 carbon atoms, and R³ and R⁴ are preferably hydrogen atoms.

The preparation processes of the amide derivatives (1) are described in detail in Japanese Patent Application Laid-Open Nos. 228048/1987 and 216852/1988. etc.

The amphipathic lipids of the component (a) may be used either singly or in any combination thereof and are incorporated in a proportion of 5 to 40 % by weight, preferably 10 to 30 % by weight in the dispersion (A).

The surfactant of the component (b) used in the dispersion (A) includes at least one selected from those commonly used for cosmetic compositions, for example, nonionic surfactants, anionic surfactants and amphoteric surfactants. Among these, examples of the nonionic surfactants include alkyl polyglycosides, polyoxyalkylene alkyl (or alkenyl) ethers, polyoxyalkylene sorbitan fatty acid esters, sorbitan fatty acid esters, polyoxyalkylene sorbitol fatty acid esters, polyoxyalkylene glycerol fatty acid esters, polyglycerol fatty acid esters, fatty acid monoglycerides, polyethylene glycol fatty acid esters and fatty acid alkanolamides.

Examples of the anionic surfactants include polyoxyalkylene alkyl ether acetic acids or salts thereof, N-acylamino acid salts, polyoxyalkylene alkyl ether sulfates, polyoxyalkylene alkyl ether phosphates, alkyl phosphates, N-acylmethyltaurine salts, alkyl sulfosuccinates, polyoxyalkylene alkyl sulfosuccinates and fatty acid salts.

Examples of the amphoteric surfactants include alkylaminoacetic acid betaines, alkylamine oxides, alkylamidopropylbetaines, alkylhydroxysulfobetaine and amidoamino acids (imidazoline type betaines).

Among these, nonionic surfactants and polyoxyalkylene alkyl ether acetic acids or salts thereof may be mentioned as preferred surfactants. The nonionic surfactant is preferably selected from the alkyl polyglycosides, polyoxyalkylene alkyl (or alkenyl) ethers, polyoxyalkylene sorbitan fatty acid esters and sorbitan fatty acid esters.

Particularly preferred nonionic surfactants includes alkyl polyglycosides, for example, those the alkyl group of which has 8 to 14 carbon atoms and the condensation degree of saccharide (glucose) of which is 1 to 2; polyoxyalkylene alkyl (alkenyl) ethers, for example, those the alkyl or alkenyl group of which has 8 to 18 carbon atoms and the average number of moles of ethylene oxide added to which is 4 to 25, preferably 4 to 15; polyoxyalkylene sorbitan fatty acid esters, for example, those the fatty acid of which has 8 to 20 carbon atoms and the average number of moles of ethylene oxide added to which is 5 to 25; and sorbitan fatty acid esters, for example, monoesters of fatty acids having 8 to 20 carbon atoms. The polyoxyethylene alkyl ether acetic acids or salts thereof include acids themselves the alkyl group of which has 8 to 20 carbon atoms and the average number of moles of ethylene oxide added to which is 3 to 15, or their salts with an alkali metal or the like. More specifically, the polyoxyethylene alkyl ether acetic acid may be used without neutralizing it as it is, or may be used after suitably neutralized. A cationic surfactant may be further added, thereby enhancing the adsorptivity of the amphipathic lipid on the skin and hair.

The surfactants of the component (b) may be used either singly or in any combination thereof and are incorporated in a proportion of 2 to 55 % by weight, preferably 5 to 40 % by weight.

In the present invention, a mixing ratio of the amphipathic lipid of the component (a) to the surfactant of the component (b) is preferably 90/10 to 25/75, more preferably 80/20 to 30/70, particularly preferably 70/30 to 40/60 in terms of a weight ratio, (a)/(b).

The dispersion (A) can be prepared by, for example, heating a mixture of the amphipathic lipid of the component (a), the surfactant of the component (b) and water to a temperature not less than the melting point of the amphipathic lipid, preferably from the melting point to (the melting point + about 10°C) with stirring to prepare a melt and cooling the melt (generally, to room temperature) with stirring after the melt is stabilized, thereby crystallizing the component (a). After this manner, a stable dispersion (A), in which the amphipathic lipid having an average particle diameter of 1 to 150 µm, preferably 1 to 80 µm, more preferably 1 to 50 µm, particularly 1 to 30 µm, most preferably 5 to 30 µm has been uniformly dispersed, is obtained. Incidentally, the term "average particle diameter" as used herein means an arithmetic mean value determined by taking a photograph of the dispersion through an optical microscope under transmission light, optionally selecting 30 particles on the photograph and measuring the longest portion in linear distance of each particle.

The particle diameter of the component (a) in the dispersion can be controlled by adjusting the kind and amount of the surfactant, cooling rate, stirring speed, etc. A publicly known pearly luster-imparting agent such as ethylene glycol distearate or ethylene glycol monostearate, and an emulsifier may also be added to the dispersion (A) before or after the process of the heating, melting and cooling.

In the dispersion (A) obtained in the above-described manner, the component (a) is stably dispersed. This dispersion may be incorporated into a cosmetic composition of the type that it is not washed off after applied to a user's body, thereby enhancing the moisturizing effect and skin roughness-improving effect of the component (a) and moreover improving its feeling upon use.

Examples of the cosmetic composition, which is used in a cosmetic method, wherein the cosmetic composition is not washed off after having been applied to a user's body, include skin and hair cosmetic compositions such as creams, emulsions, lotions, oily cosmetic compositions, gel, essences, lip sticks, foundations, hair tonics, hair styling compositions, hair grooming compositions and hair growth stimulants.

The amount of the dispersion (A) incorporated into the cosmetic composition, which is used in a cosmetic method, wherein the cosmetic composition is not washed off after applied to a user's body, may be suitably adjusted according to the kind of the cosmetic composition and the concentration of the dispersion (A), and the dispersion (A) is preferably incorporated in such an amount that the amphipathic lipid is contained in a proportion of generally 0.01 to 30 % by weight, particularly 0.1 to 20 % by weight.

As the amphipathic lipid used in the cosmetic composition, the amide derivative (1) is particularly preferred like the component (a) in the dispersion. The amphipathic lipids may be used either singly or in any combination thereof. The average particle diameter thereof must be within a range of 1 to 150 µm from the viewpoint of a feel, and is preferably 1 to 80 µm, more preferably 1 to 50 µm, particularly 1 to 30 µm, most preferably 5 to 30 µm. Incidentally, the term "average particle diameter" as used herein means a measured value according to the above-described method. The amphipathic lipid is incorporated in a proportion of 0.01 to 30 % by weight, preferably 0.1 to 20 % by weight in the cosmetic composition used in the cosmetic method, wherein the composition is not washed off according to the present invention.

Besides the above-described essential component, ingredients commonly incorporated in cosmetic compositions, quasi-drugs, drugs and the like, for example, oils, water, surfactants, silicones, pH adjusters, inorganic salts, viscosity modifiers, medicinally-effective ingredients, other moisturizers, chelating agents, ultraviolet light absorbents, preservatives, colorants, perfume bases and the like, may be incorporated in the cosmetic compositions so far as no detrimental influence is thereby imposed on the effects of the present invention.

As the oils, may be used, for example, hydrocarbons such as vaseline, solid and liquid paraffins, crystal oil, ceresin, ozocerite, montan wax, squalane and squalene; ester oils such as olive oil, carnauba wax, lanolin, jojoba oil, glyceryl monostearate, glyceryl monooleate, isopropyl stearate and neopentyl glycol dicaprate; higher fatty acids such as stearic acid and palmitic acid; higher alcohol such as cetanol and stearyl alcohol; and silicone oil.

When these oils are incorporated, they may preferably be preferably incorporated in a proportion of 0.01 to 50 % by weight, particularly 0.1 to 30 % be weight based on the cosmetic composition.

Water is preferably used as a base material together with a water-soluble alcohol. In particular, the use of water together with ethanol and a water-soluble polyhydric alcohol is preferred because more enhanced water-retaining ability is achieved. No particular limitation is imposed on the water-soluble polyhydric alcohol so far as it has at least two hydroxyl groups in its molecule, and examples thereof include ethylene glycol, propylene glycol, 1,3-butylene glycol, 1,4-butylene glycol, dipropylene glycol, glycerol, polyglycerols such as diglycerol, triglycerol and tetraglycerol, glucose, maltose, maltitol, sucrose, fructose, xylitol, sorbitol, maltotriose, threitol, erythritol, and alcohols obtained by reduction of amylolytic sugar. These alcohols may be used either singly or in any combination thereof. The amount of the alcohols incorporated into the cosmetic composition may be suitably selected according to the form of the cosmetic composition, but they are preferably incorporated in such a manner that the total amount thereof amounts to 0.01 75 % by weight, particularly 0.1 to 60 % by weight.

Examples of the surfactants include polyoxyethylene alkyl ethers, polyoxyethylene fatty acid esters, polyoxyethylene sorbitan fatty acid esters, glycerol fatty acid esters, polyoxyethylene hardened castor oil, alkylsulfates, polyoxyethylene alkylsulfates, alkylphosphates, polyoxyethylene alkylphosphates, alkali metal salts of fatty acids, sorbitan fatty acid esters, glycerol fatty acid esters and alkyl glyceryl ethers. These surfactants may be used either singly or in any combination thereof. When these surfactants are incorporated, it is preferable to incorporate them in a proportion of 0.01 to 50 % by weight, particularly 0.1 to 30 % by weight based on the total weight of the cosmetic composition.

No particular limitation is imposed on the silicones so far as they are commonly incorporated in the cosmetic compositions, and, for example, octamethylpolysiloxane, tetradecamethylpolysiloxane, methylpolysiloxane, high-polymeric methylpolysiloxane and methylphenylpolysiloxane, and besides methylpolycycloxanes such as octamethylcyclotetrasiloxane and decamethylcyclopentasiloxane, and trimethylsiloxysilicic acid may be used as oil. Further, modified silicones such as polyether-modified silicones, polyether and alkyl-modified silicones, alkyl glyceryl ether-modified silicones may be used as surfactants. When these silicones are incorporated, the amount of the silicones incorporated into the cosmetic composition may be controlled in such a manner that the total amount of the silicones and the oils or surfactants other than the silicones amounts to the above-described incorporating amount.

No particular limitation is imposed on the pH adjusters. However, examples thereof include metal hydroxides such as sodium hydroxide, potassium hydroxide and lithium hydroxide, triethanolamine, isopropanolamine, diisopropanolamine, urea, ε-aminocarponic acid, sodium pyrrolidone carboxylate, sodium hydrogenphosphate, sodium citrate, organic acids such as citric acid, lactic acid, succinic acid and tartaric acid, and betaines such as glycinebetaine and lysinebetaine. The cosmetic compositions are preferably adjusted to a pH within a range of 3.5 to 10. When the cosmetic composition is used for xerosis, atopic xeroderma and the like, the pH is preferably controlled to an acid to neutral range of 4.5 to 7.5.

Examples of the inorganic salts include magnesium sulfate, potassium sulfate, sodium sulfate, magnesium chloride and sodium chloride.

Examples of the viscosity modifiers include polyvinyl alcohol, carboxyvinyl polymers, carboxymethyl cellulose, gelatin, tragacanth gum, xanthan gum, hyaluronic acid, agarose and sodium alginate.

The medicinally-effective ingredients include antiphlogistics such as dipotassium glycyrrhizinate and allantoin; and germicides and antidandruff agents such as benzalkonium chloride, benzalkonium cetylphosphate (Kao Corporation; Sanizol P), triclosan, triclocarban, octopilox and zinc pyrithione.

Upon preparation of the cosmetic composition, the amphipathic lipid may be used as a dispersion (A) prepared in advance or as it is. When the amphipathic lipid is used as it is, it is preferably adjusted to a specified particle diameter (1 to 150 µm, preferably 1 to 80 µm, more preferably 1 to 50 µm, particularly 1 to 30 µm, most preferably 5 to 30 µm in terms of an average particle diameter) by grinding or the like and mixed with other cosmetic components. When the amphipathic lipid is used as the dispersion (A) prepared in advance on the other hand, it is prepared that the cosmetic components other than the dispersion (A) be mixed with each other in advance, and the mixture be mixed with the dispersion (A) at not more than 50°C to prepare a cosmetic composition. By following this process, the amphipathic lipid in the dispersion (A) is stably incorporated into the cosmetic composition.

### EXAMPLES

### Preparation Example 1 of dispersion:

A mixture of an amphipathic lipid [in the general formula (1), R¹ = C₁₅H₃₁, R² = C₁₆H₃₃, R³ = H, R⁴ = H; melting point: 74 to 76°C; 15 parts by weight], Midol 10 [active component: 40 % by weight; decyl polyglycoside (condensation degree: 1 to 1.35); product of Kao Corporation; 25 parts by weight] and water (60 parts by weight) was heated to 80 to 85°C and then cooled with stirring to crystallize the amphipathic lipid. The mixture was further cooled to room temperature (25°C) with stirring to prepare a dispersion of the amphipathic lipid.

The particles of the amphipathic lipid in the dispersion thus obtained are needle crystals having an average particle diameter of 15.1 µm.

### Preparation Examples 2 to 7 of dispersion:

Their corresponding dispersions of amphipathic lipids shown in Table 1 were prepared in the same manner as in Preparation Example 1 of dispersion.

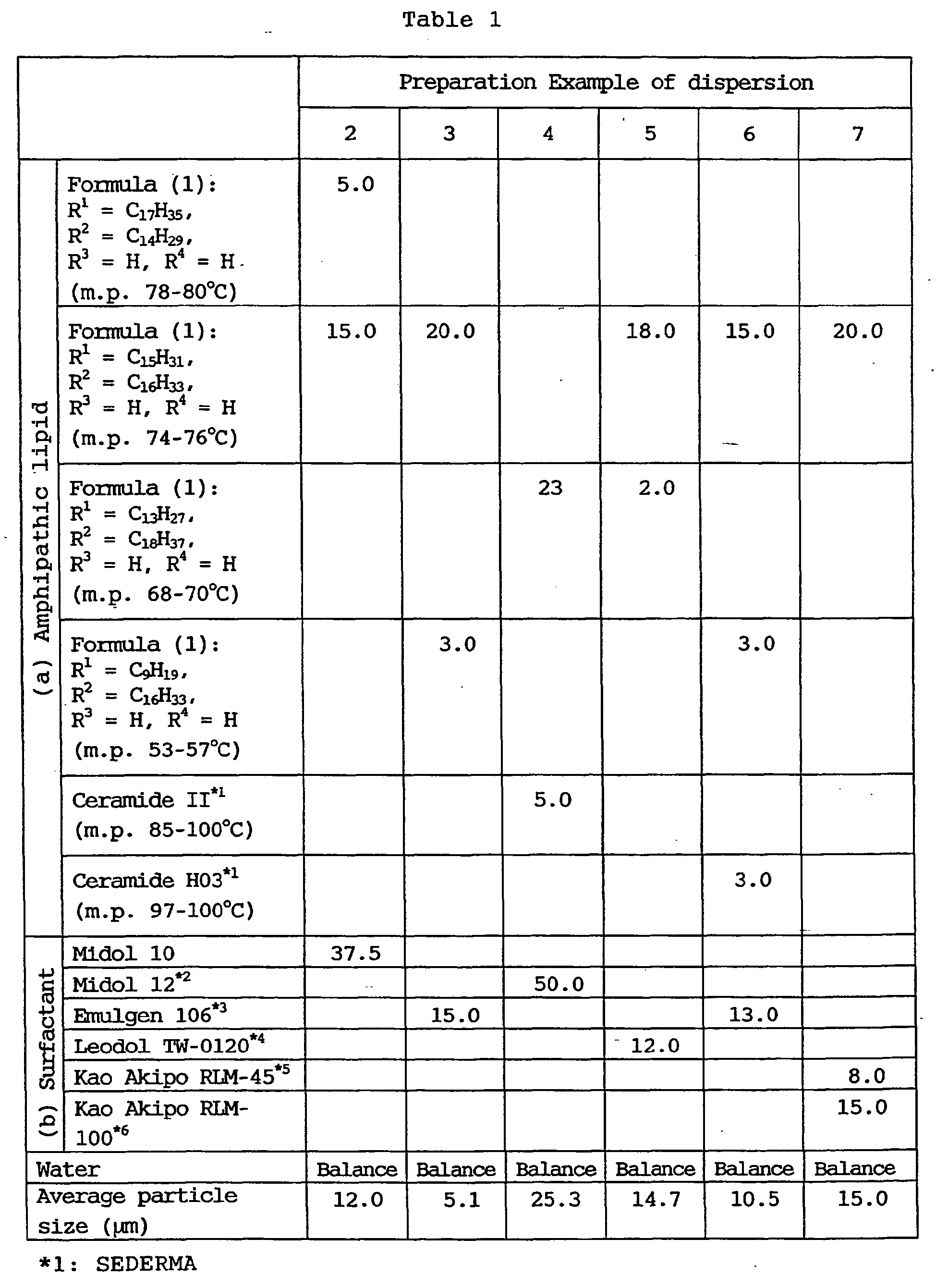

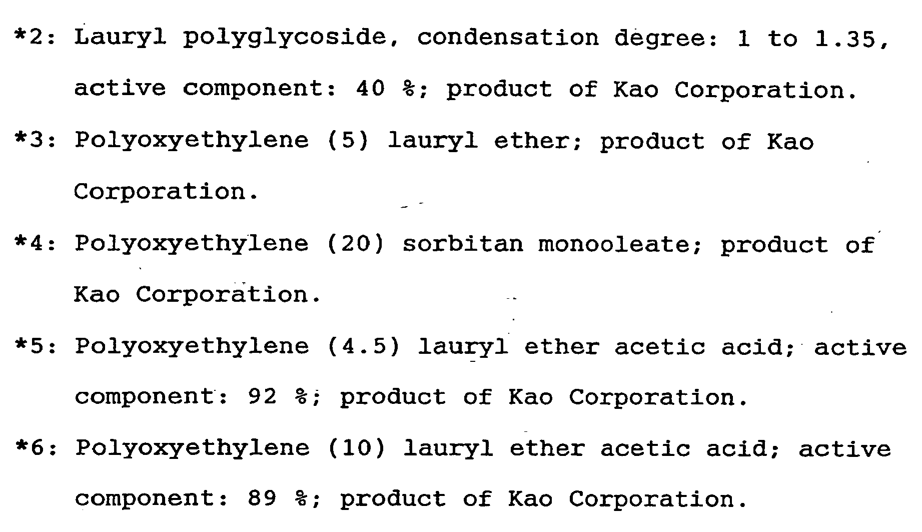

### Example 1: (O/W lotion)

| | | (% by weight) |
|---|---|---|
| (1) | Cholesterol | 1.0 |
| (2) | Stearic acid | 0.6 |
| (3) | Palmitic acid | 0.4 |
| (4) | Cholesteryl isostearate | 1.0 |
| (5) | Glyceryl isostearate | 1.0 |
| (6) | Squalane | 3.0 |
| (7) | Cetanol | 4.8 |
| (8) | Stearyl alcohol | 3.2 |
| (9) | Isopropyl palmitate | 1.8 |
| (10) | Sorbitan monostearate | 1.1 |
| (11) | Methyl polysiloxane | 1.0 |
| (12) | 86% Glycerol | 5.0 |
| (13) | Ethanol | 2.0 |
| (14) | Water | Balance |
| (15) | Dispersion of amphipathic lipid | 20.0 |
| | (Preparation Example 1) | |
| | Total | 100.0 |

(1) to (11) were heated to 65°C to melt it, and (12) to (14) were added to the melt and uniformly mixed to prepare an O/W lotion. However, (15) the dispersion (Preparation Example 1) in which an amphipathic lipid had been crystallized in advance was added lately at 40°C.

### Example 2: (O/W cream)

An O/W cream having a composition shown below was prepared in a method known *per se* in the art. However, the dispersion (Preparation Example 5) in which an amphipathic lipid had been crystallized in advance was added lately at 40°C.

| | | (% by weight) |
|---|---|---|
| (1) | Polyoxyethylene (10) hardened castor oil | 1.0 |
| (2) | Sorbitan monostearate | 0.5 |
| (3) | Sodium stearoylmethyltaurine | 0.5 |
| (4) | Cetostearyl alcohol | 2.0 |
| (5) | Stearic acid | 1.8 |
| (6) | Cholesterol | 1.5 |
| (7) | Cholesteryl isostearate | 1.0 |
| (8) | Neopentyl glycol dicaprate | 8.0 |
| (9) | Methylpolysiloxane | 5.0 |
| (10) | Glycerol | 5.0 |
| (11) | Purified water | Balance |
| (12) | Dispersion of amphipathic lipid | 25.0 |
| | (Preparation Example 5) | |
| | Total | 100.0 |

### Comparative Example 1:

A lotion was prepared by removing the amphipathic lipid from the lotion in Example 1 and balancing it by water.

### Comparative Example 2:

A cream was prepared by removing the amphipathic lipid from the cream in Example 2 and balancing it by water.

### Test Example 1: Test for skin roughness-improving effect

Chosen as a group of subjects in the dry season of winter were 3 women and 3 men (6 persons in total) who had felt their own skin roughness, itch or dryness on their bodies, and 5 groups were provided (30 persons in total). Proper amounts of different samples (the lotion in Example 1, the cream in Example 2, the lotion in Comparative Example 1, the cream in Comparative Example 2 and untreated) were applied to a leg of each subject in respective groups once a day for 2 weeks (not applied to the untreated group). On the following day of the completion of the 2-week application test, the skin roughness-improving effect of each sample (on the leg) was visually observed after the subjects were allowed to rest for 20 minutes in a room which was air-conditioned at 20°C and 40% humidity, thereby ranking the samples in accordance with the following standard. The results are shown as an average score of 6 persons in Table 2 (Evaluation standard for visual ranking (scaling) of skin roughness) -
3: Clearly improved;
2: Slightly improved;
1: Not changed; and
0: Worsened.

**Table 2**

| | Lotion and cream of the invention | | Comparative lotion and cream | | Untreated |
|---|---|---|---|---|---|
| | 1 | 2 | 1 | 2 | |
| Skin roughness | 2.3 | 2.5 | 1.5 | 1.8 | 1.0 |

It was understood from the results that the inventive products can significantly improve skin roughness compared with the comparative products.

### Test Example 2:

### Recovery effect on model roughened skin (measurement of skin conductance)

A cap was attached to the inner side (the side to be bent) of the lower arm of each of subjects (10 persons in total of 5 women and 5 men) to conduct a treatment (for 30 minutes) with acetone/ether, thereby providing model roughened skins. Proper amounts of the lotions of Example 1 and Comparative Example 1 were respectively applied to the model roughened skins twice a day for 5 days. After 5 days, the recovery effects of the lotions on the model roughened skins were evaluated.
The evaluation was conducted by measuring the skin conductance of each model roughened skin by means of a skin conductance meter (manufactured by IBS Company). The results are indicated by an average value of 10 persons. A higher skin conductance value indicates that the test sample has a higher recovery effect. As a result, it was understood that the inventive product has a high recovery effect on the roughened skin compared with the comparative.

**Table 3**

| | Example 1 | Comparative Ex. 1 |
|---|---|---|
| Skin conductance (µ-siemens) | 34.3 | 22.7 |

### Example 3: (O/W cream)

An O/W cream having a composition shown below was prepared in a method known *per se* in the art. However, the dispersion (Preparation Example 3) in which an amphipathic lipid had been crystallized in advance was added lately at 40°C.

| | | (% by weight) |
|---|---|---|
| (1) | Stearic acid | 0.7 |
| (2) | Myristic acid | 0.3 |
| (3) | Squalane | 3.0 |
| (4) | Olive oil | 3.0 |
| (5) | paraffin | 1.5 |
| (6) | Behenyl alcohol | 4.0 |
| (7) | Self-emulsifying glyceryl monostearate | 1.0 |
| (8) | Sorbitan monostearate | 3.0 |
| (9) | Glycerol | 10.0 |
| (10) | Liquid sorbit | 10.0 |
| (11) | Water | Balance |
| (12) | Amphipathic lipid | 5.0 |
| | (Preparation Example 3) | |
| | Total | 100.0 |

The thus-obtained cream was a cream that did not irritate and had an excellent feel and an excellent moisturizing effect.

### Example 4: (Ointment)

An ointment having a composition shown below was prepared in a method known *per se* in the art. All the components were mixed at the same time due to preparation at ordinary temperature.

| | | (% by weight) |
|---|---|---|
| (1) | Paraffin | 10.0 |
| (2) | White Vaseline | 82.0 |
| (3) | Beeswax | 3.0 |
| (4) | Amphipathic lipid | 5.0 |
| | (Preparation Example 2) | |
| | Total | 100.0 |

The thus-obtained ointment was an ointment that did not irritate and had an excellent moisturizing effect.

### Example 5: (Separation type toilet lotion)

A toilet lotion having a composition shown below was prepared in a method known *per se* in the art. However, the dispersion (Preparation Example 7) in which an amphipathic lipid had been crystallized in advance was added lately at 30°C.

| | | (% by weight) |
|---|---|---|
| (1) | Ethanol | 2.0 |
| (2) | Glycerol | 10.0 |
| (3) | Zinc oxide | 0.5 |
| (4) | Talc | 1.0 |
| (5) | Water | Balance |
| (6) | Amphipathic lipid | 5.0 |
| | (Preparation Example 7) | |
| | Total | 100.0 |

The thus-obtained toilet lotion was a lotion that did not irritate and had an excellent feel and an excellent moisturizing effect.

### INDUSTRIAL APPLICABILITY

The cosmetic compositions used in a cosmetic method wherein they are not washed off after applied to user's bodies are excellent in moisturizing effect and skin roughness-improving effect and give users a pleasant feeling upon use.

## Claims

1. Use of a cosmetic composition comprising a dispersion (A) containing the following components (a) and (b) :
(a) 5 to 40 % by weight of a dispersoid composed of a particulate amphipathic lipid having at least one hydroxyl and amide group in its molecule, which is solid at 25 °C, and which has an average particle diameter of 1 to 150 µm; and
(b) 2 to 55 % by weight of a surfactant in an aqueous medium,
in a cosmetic method, wherein said cosmetic composition is applied to a user's body, and wherein said cosmetic composition is not washed off from said user's body after said cosmetic composition has been applied thereto.

2. Use according to claim 1, wherein (b) the surfactant is at least one selected from nonionic surfactants composed of alkyl polyglycosides, polyoxyalkylene alkyl (or alkenyl) ethers, polyoxyalkylene sorbitan fatty acid esters and sorbitan fatty acid esters, and polyoxyethylene alkyl ether acetic acids or salts thereof.

3. Use of a cosmetic composition comprising 0.01 to 30 % by weight of a particulate amphipathic lipid having an average particle diameter of 1 to 150 µm, which is solid at 25 °C, and which has at least one hydroxyl and amide group in its molecule,
in a cosmetic method, wherein said cosmetic composition is applied to a user's body, and wherein said cosmetic composition is not washed off from said user's body after said cosmetic composition has been applied thereto.

4. Use according to claim 3, wherein the cosmetic composition further comprises a surfactant.

5. Use of a cosmetic composition according to any one of Claims 1 to 4, wherein the amphipathic lipid is a ceramide or a ceramide analogue.

## Patentansprüche

1. Verwendung einer Kosmetikzusammensetzung, umfassend eine Dispersion (A) mit den folgenden Komponenten (a) und (b):
(a) 5 bis 50 Gew.% eines Dispersoids, das sich zusammensetzt aus einem körnigen amphipathischen Lipid mit zumindest einer Hydroxyl- und Amidgruppe im Molekül, das ein Feststoff bei 25°C ist und einen durchschnittlichen Teilchendurchmesser von 1 bis 150 µm aufweist; und
(b) 2 bis 55 Gew.% eines Tensids in einem wäßrigen Medium,
in einem kosmetischen Verfahren, wobei die Kosmetikzusammensetzung auf den Körper des Benutzers aufgetragen wird und wobei die Kosmetikzusammensetzung vom Körper des Benutzers nach Auftragen der Kosmetikzusammensetzung nicht abgewaschen wird.

2. Verwendung nach Anspruch 1, worin (b) das Tensid zumindest eines ist ausgewählt aus nicht-ionischen Tensiden, die sich zusammensetzen aus Alkylpolyglycosiden, Polyoxyalkylenalkyl- (oder -alkenyl-) -ethern, Polyoxyalkylensorbitanfettsäureestern und Sorbitanfettsäureestern und Polyoxyethylenalkyletheressigsäuren oder Salzen davon.

3. Verwendung einer Kosmetikzusammensetzung, umfassend 0,01 bis 30 Gew.% eines teilchenförmigen amphipathischen Lipids mit einem durchschnittlichen Teilchendurchmesser von 1 bis 150 µm, das bei 25°C fest ist und zumindest eine Hydroxyl- und Amidgruppe im Molekül aufweist, in einem kosmetischen Verfahren, wobei die Kosmetikzusammensetzung auf den Körper eines Benutzers aufgetragen wird und wobei die Kosmetikzusammensetzung vom Körper des Benutzers nach Auftragen der Kosmetikzusammensetzung nicht abgewaschen wird.

4. Verwendung nach Anspruch 3, worin die Kosmetikzusammensetzung weiterhin ein Tensid umfaßt.

5. Verwendung einer Kosmetikzusammensetzung nach einem der Ansprüche 1 bis 4, worin das amphipathische Lipid ein Ceramid oder Ceramidanalog ist.

## Revendications

1. Utilisation d'une composition cosmétique comprenant une dispersion (A) contenant les composants (a) et (b) suivants :
(a) 5 à 40 % en poids d'un dispersoïde composé d'un lipide amphipathique particulaire ayant au moins un groupe hydroxyle et amide dans sa molécule, qui est solide à 25°C et qui présente un diamètre particulaire moyen de 1 à 150 µm ; et
(b) 2 à 55 % en poids d'un agent tensioactif dans un milieu aqueux,
dans un procédé cosmétique, dans lequel ladite composition cosmétique est appliquée au corps de l'utilisateur, et dans lequel ladite composition cosmétique n'est pas enlevée par lavage dudit corps de l'utilisateur après que ladite composition cosmétique y ait été appliquée.

2. Utilisation selon la revendication 1, dans laquelle (b) l'agent tensioactif est au moins un des agents tensioactifs non ioniques composés de alkyl polyglycosides, d'éthers de polyoxyalkylène alkyl (ou alcényle), esters d'acides gras polyoxyalkylène sorbitan et esters d'acides gras sorbitan, et acides acétiques d'éther polyoxyéthylène alkyl ou leurs sels.

3. Utilisation d'une composition cosmétique comprenant 0,01 à 30 % en poids d'un lipide amphipathique particulaire ayant un diamètre particulaire moyen de 1 à 150 µm, qui est solide à 25°C et qui possède au moins un groupe hydroxyle et amide dans sa molécule,
dans un procédé cosmétique, dans lequel ladite composition cosmétique est appliquée au corps de l'utilisateur, et dans lequel ladite composition cosmétique n'est pas enlevée par lavage du corps dudit utilisateur après que ladite composition cosmétique y ait été appliquée.

4. Utilisation selon la revendication 3, dans laquelle la composition cosmétique comprend en outre un agent tensioactif.

5. Utilisation d'une composition cosmétique selon l'une quelconque des revendications 1 à 4, dans laquelle le lipide amphipathique est un céramide ou un analogue du céramide.
